# EUROPÄISCHE PATENTSCHRIFT

(11) **EP 4 225 537 B1**
(45) Veröffentlichungstag und Bekanntmachung des Hinweises auf die Patenterteilung: **03.12.2025**
(21) Anmeldenummer: 22801770.3
(22) Anmeldetag: 12.10.2022
(51) Int. Cl.: B25J 9/16

(54) **KALIBRIERUNGSVERFAHREN ZUR AUTOMATISIERTEN KALIBRIERUNG VON KAMERA ZU MEDIZINISCHEM ROBOTER UND CHIRURGISCHES ASSISTENZSYSTEM**
CALIBRATION METHOD FOR THE AUTOMATED CALIBRATION OF A CAMERA WITH RESPECT TO A MEDICAL ROBOT, AND SURGICAL ASSISTANCE SYSTEM
PROCÉDÉ D'ÉTALONNAGE POUR L'ÉTALONNAGE AUTOMATISÉ D'UNE CAMÉRA PAR RAPPORT À UN ROBOT MÉDICAL, ET SYSTÈME D'ASSISTANCE CHIRURGICALE

(30) Priorität: 13.10.2021 DE 102021126484
(43) Veröffentlichungstag der Anmeldung: 16.08.2023
(73) Patentinhaber: B. Braun New Ventures GmbH, 79110 Freiburg im Breisgau (DE)
(72) Erfinder: BEYL, Tim, 76532 Baden-Baden (DE)
(74) Vertreter: Winter, Brandl - Partnerschaft mbB
(86) Internationale Anmeldenummer: PCT/EP2022/078423
(87) Internationale Veröffentlichungsnummer: WO 2023/062091

(56) Entgegenhaltungen:
- WO-A1-2021/087433
- OZGUNER ORHAN ET AL: "Camera-Robot Calibration for the Da Vinci Robotic Surgery System", IEEE TRANSACTIONS ON AUTOMATION SCIENCE AND ENGINEERING, IEEE SERVICE CENTER, NEW YORK, NY, US, vol. 17, no. 4, 1 October 2020 (2020-10-01), pages 2154 - 2161, XP011813394, ISSN: 1545-5955, [retrieved on 20201005], DOI: 10.1109/TASE.2020.2986503

## Beschreibung

### Technisches Gebiet

Die vorliegende Offenbarung betrifft ein Kalibrierungsverfahren zur automatisierten Kalibrierung einer Roboterkamera zu bzw. gegenüber einem medizinischen, insbesondere chirurgischen, Roboter und zur automatisierten Kalibrierung eines externen Kamerasystems mit zumindest einer externen Kamera zu dem Roboter. Die Roboterkamera ist dabei an einem Roboterarm des Roboters, der an eine Roboterbasis angebunden /verbunden ist, gegenüber der Roboterbasis beweglich geführt. Daneben betrifft die vorliegende Offenbarung ein chirurgisches navigiertes Assistenzsystem/ einen navigierten robotischen Manipulator sowie ein computerlesbares Speichermedium gemäß den Oberbegriffen der nebengeordneten Ansprüche.

### Technischer Hintergrund der Offenbarung

Ein typisches Problem im Bereich der medizinischen Robotik, insbesondere der chirurgischen Robotik, ist die sogenannte Hand-Auge-Kalibrierung (hand-eye-calibration), bei der eine Transformation zwischen einer Kamera und einem Roboter gesucht wird, um gewissermaßen die Welt der Erfassung bzw. Sicht und die Welt der Roboterkinematik aufeinander abzustimmen und miteinander in Verbindung zu bringen.

Es gibt bei dieser Hand-Augen-Kalibrierung typischerweise zwei Fälle bzw. Szenarien, wobei in einem ersten Fall eine Kamera von dem Roboter direkt gehalten und geführt wird, eine sogenannte Auge-in-Hand/ eye-in-hand Konfiguration, und in einem zweiten Fall eine (externe) Kamera extern, insbesondere statisch, angeordnet ist und den bewegbaren Roboter selbst oder einen Tracker/ einen Marker nachverfolgt, der vom Roboter gehalten wird (sogenannte Auge-an-Basis/ eye-on-basis Konfiguration).

Der Zweck dieser Kalibrierung besteht, wie vorstehend angedeutet, darin, die Aufnahmeverarbeitung der Kamera mit der Roboterkinematik zu kombinieren bzw. zu verknüpfen, so dass etwa Objekte im Sichtfeld der Kamera, insbesondere mittels Bildverarbeitung bzw. Bildanalyse, identifiziert werden können und ein Plan für den Roboter zum Ergreifen bzw. Manipulieren dieser Objekte erstellt werden kann. Insbesondere kann während eines medizinischen Eingriffs der Roboter mittels eines Endeffektors und einer durch die Kamera bereitgestellte Aufnahme eine Manipulation eines Gewebes eines Patienten durchführen. Daneben gibt es noch weitere medizinische Szenarien, in denen eine Hand-Augen-Kalibrierung/ hand-eye-calibration erforderlich ist.

In der Regel ist neben der Hand-Augen-Kalibrierung noch eine weitere Kalibrierung erforderlich, nämlich eine sogenannte geometrische Kalibrierung der Kamera selbst, um optische Parameter wie etwa die Brennweite der Kamera zu ermitteln. Diese geometrische Kalibrierung ist erforderlich, um mit einer eingesetzten Kamera geometrisch korrekte Darstellungen der beobachteten Szene zu erzeugen und eine präzise Manipulation durch den Roboter zu gewährleisten. Für die geometrische Kamerakalibrierung wird in der Regel ein Kalibrierungsmuster benötigt. Das Kalibrierungsmuster muss im Sichtfeld der Kamera platziert werden, und die Kalibrierung erfolgt mit Hilfe der bekannten Geometrie des Musters und eines Kalibrierungsalgorithmus, welcher Kameraparameter wie Brennweite oder Verzeichnungskoeffizienten aus den beobachteten Aufnahmen und Verzerrungen errechnet. Das Kalibrierungsmuster muss dabei relativ zu der Kamera bewegt werden, entweder durch Verschieben des Kalibrierungsmusters selbst oder durch Bewegen der Kamera.

Insbesondere sind beide Kalibrierungen erforderlich, um einen medizinischen Roboter zusammen mit einer Kamera, sei diese extern zu dem Roboter angeordnet (hand-on-base) oder an dem Roboter selbst (Roboterkamera), insbesondere im Bereich eines Endeffektors, zu verwenden.

Zwar gibt es im Stand der Technik Methoden für eine Hand-Augen-Kalibrierung (hand-eye calibration) und eine geometrische Kalibrierung, jedoch sind diese durch einen Nutzer händisch/ manuell gemäß einem Kalibrierungsschema durchzuführen und sehr aufwändig. Zudem sind diese Methoden aufgrund der manuellen Handhabung fehleranfällig und bieten oftmals nicht die notwendige Präzision der Kalibrierung, insbesondere wenn ein medizinischer Roboter bei einem chirurgischen Eingriff mit Toleranzen von wenigen Millimetern eingesetzt, und vor dem Eingriff selbst die Kalibrierung durchgeführt werden soll.

Beispielsweise offenbart der wissenschaftliche Artikel "Camera-Robot Calibration for the Da Vinci Robotic Surgery System" IEEE Service Center, New York, Bd. 14, Nr. 4 eine Methode für autonome oder halbautonome Roboter mit teleoperativen Geräten, welche eine präzise Hand-Augen-Kalibrierung zwischen einer frei beweglichen Endoskopkamera und einem patientenzugewandten Manipulatorarm (PSM) erfordert. Zunächst wird hierbei eine Reihe an Bildverarbeitungsschritten sowie optische Trackingoperationen durchgeführt, um erforderliche Koordinatentransformationen zwischen einem Bild der endoskopischen Kamera und dem Kameragehäuse zu erhalten. Danach werden kinematische Eigenschaften des Roboters ausgenutzt, um Koordinatentransformationen zwischen zwei KOS zu berechnen.

Ferner offenbart die WO 2021/087433 A1 ein robotergestütztes chirurgisches Navigationssystem mit einer stereoskopischen Kamera. In diesem Navigationssystem werden eine Vielzahl von Transformationen berechnet, um schließlich einen Sichtvektor der stereoskopischen Kamera in einem KOS des Roboters zu bestimmen, um so eine Bewegung des Roboterarms auf der Grundlage von Befehlen ermöglicht, die von einem Benutzer in Bezug auf den Sichtvektor der Kamera gegeben werden.

### Zusammenfassung der vorliegenden Offenbarung

Es sind daher die Aufgaben und Ziele der vorliegenden Offenbarung, die Nachteile aus dem Stand der Technik zu vermeiden oder zumindest zu mindern und insbesondere ein Kalibrierungsverfahren, ein chirurgisches Assistenzsystem/ einen navigierten robotischen Manipulator, sowie ein computerlesbares Speichermedium bereitzustellen, welches automatisiert, also ohne notwendige manuelle Eingaben eines Nutzers, besonders einfach, schnell, effizient und fehlerfrei eine Kalibrierung/ Registrierung/ Verknüpfung zwischen einem Roboter und einer Kamera bereitstellt. Eine weitere Teilaufgabe besteht darin, mit möglichst wenigen und insbesondere standardisierten medizinischen Geräten, eine solche Kalibrierung durchführen, um in einem Operationssaal möglichst keine zusätzlichen zu den während der Operation eingesetzten Geräte vorhalten zu müssen und um Kosten niedrig zu halten. Eine weitere Teilaufgabe ist, dem Nutzer, insbesondere dem Chirurgen, eine intuitive und automatisierte Kalibrierung bereitzustellen, so dass der Nutzer, insbesondere ein medizinisches Fachpersonal, keine besondere Schulung einer Kalibrierung benötigt.

Die Aufgaben werden hinsichtlich eines gattungsgemäßen Kalibrierungsverfahrens/ Registrierungsverfahrens erfindungsgemäß durch die Merkmale des Anspruchs 1 gelöst, hinsichtlich eines gattungsgemäßen chirurgischen Assistenzsystems erfindungsgemäß durch die Merkmale des Anspruchs 8 gelöst und hinsichtlich eines computerlesbaren Speichermediums erfindungsgemäß durch die Merkmale des Anspruchs 12 gelöst.

Vorliegend wird also ein automatisiertes Kalibrierungsverfahren/ Registrierverfahren sowie ein automatisiertes/ automatisches Kalibrierungssystembereitgestellt, das eine Kalibrierung zwischen einer an einem Roboter montierten Kamera (Roboterkamera) und einem (externen) Kamerasystem, das den Roboter beobachtet, durchführt. In der vorliegenden Konfiguration werden im Unterschied zum Stand der Technik zumindest zwei Kameras verwendet, wobei eine externe Kamera an einer Basis (eye-on-base) und eine weitere Kamera (Roboterkamera) an dem Roboter (eye-in-hand) angebracht ist, um aktiv gesteuert und bewegt zu werden. Über das automatisierte Kalibrierungsverfahren lässt sich der Vorgang der Kalibrierung automatisiert, insbesondere nacheinander/ sequentiell durchführen. Die vorliegende Offenbarung ermöglicht es, beide Kameras, also sowohl die externe Kamera als auch die Roboterkamera, zusammen mit dem Roboter zu kalibrieren bzw. zu registrieren. Damit werden beide Kameras auf den Roboter kalibriert. Durch die automatisierte Kalibrierung mit einhergehender Redundanz kann auch ein Fehler minimiert werden, etwa, wenn sich ein medizinisches Fachpersonal in das Sichtfeld zwischen der externen Kamera und dem Roboter bewegt und eine Sicht verdeckt. Hierbei kann ggf. die kalibrierte Roboterkamera die Steuerung weiterführen.

Ein Grundgedanke der vorliegenden Offenbarung liegt also darin, sowohl am Roboter selbst eine Kamera (eye-in-hand) vorzusehen, die über den Roboterarm bewegbar ist, als auch zusätzlich eine externe Kamera (eye-on-base) vorzusehen, die den Roboterarm oder einen Endabschnitt des Roboterarms für eine Nachverfolgung erfasst und zumindest einen Roboterflansch und/oder einen Tracker erfasst, wenn sich dieser in das Sichtfeld der externen Kamera bewegt. In einem ersten Schritt der automatisierten Kalibrierung wird die Roboterkamera (eye-in-hand) derart bewegt, dass diese die externe Kamera (eye-on-base) erfasst und eine geeignete Transformation zwischen der Roboterkamera (eye-in-hand) und der externen Kamera (eye-on-base) ermittelt. In einem weiteren Schritt wird dann der Roboterflansch, insbesondere ein an dem Roboterflansch angebrachter Tracker/ Markierungselement, im Sichtbereich der externen Kamera bewegt und es wird der Roboterflansch, insbesondere der Tracker, (hinsichtlich einer Lage) nachverfolgt. Die Kinematik des Roboters zwischen der Roboterbasis und dem Roboterflansch kann über die Steuerung erfasst werden und darüber kann auch eine Transformation zwischen der Roboterbasis und dem Roboterflansch bereitgestellt werden. Auf dieser Basis wird dann schließlich die Hand-Augen-Kalibrierung durchgeführt. Mit der vorliegenden Offenbarung wird eine simultane Kalibrierung/ Registrierung zwischen der Roboterkamera und dem Roboter sowie der externen Kamera und dem Roboter durchgeführt.

Diese Konfiguration ist insbesondere in einem solchen chirurgischen Szenario von Bedeutung, in dem die externe Kamera (eye-on-base) beispielsweise eine Tracking-Kamera ist, die zur Nachverfolgung des Roboters verwendet wird, der als Werkzeug in einem chirurgischen Navigationsszenario eingesetzt wird. Ebenso von Bedeutung ist natürlich auch der Fall bei dem eine optische Kamera für eine optische Aufnahme eingesetzt wird. Bei der vom Roboter gehaltenen Kamera (Roboterkamera; eye-in-hand) kann es sich ebenso insbesondere um eine Tracking-Kamera für eine Nachverfolgung oder um eine optische Kamera handeln.

Im Bereich der Medizin kann etwa die vom Roboter gehaltene Roboterkamera verwendet werden, um ein näheres Bild des Patienten zu erhalten (insbesondere für die Bildverarbeitung). Alternativ oder zusätzlich könnte die Kamera auch eine Mikroskop-Kamera sein, wobei die Roboter- und Kamerabaugruppe ein Operationsmikroskop bilden. In einem anderen medizinischen Szenario kann die robotergehaltene Kamera zum Beispiel zur Visualisierung von Objekten verwendet werden, die von der Kamera auf der Basis nicht sichtbar sind.

Mit anderen Worten umfasst ein Kalibrierungsverfahren zur automatisierten Kalibrierung einer Roboterkamera zu einem Roboter, die an einem Roboterarm mit einem Roboterflansch des Roboters, der an eine Roboterbasis angebunden /verbunden ist, beweglich geführt ist, und zur automatisierten Kalibrierung eines externen Kamerasystems mit zumindest einer externen Kamera zu dem Roboter, die Schritte: Bewegen der Roboterkamera (eye-in-hand) mittels des Roboterarms unter Erfassung und Aufnahme einer Umgebung; Detektion eines vorbestimmten optischen Kalibrierungsmusters und dessen Lage mit vorbestimmter Transformation/ Relation zu einer Lage der externen Kamera und/oder Detektion einer Lage der externen Kamera auf Basis der Aufnahme; Bestimmen, auf Basis der ermittelten Lage der externen Kamera, einer Transformation zwischen der Roboterkamera und der externen Kamera und Bestimmen eines Sichtfelds der externen Kamera; Bewegen des Roboterflansches, insbesondere mit zumindest einem am Roboterflansch angebrachten Tracker/ Markierungseinheit, in zumindest drei unterschiedliche Lagen/ Posen im (zuvor bestimmten) Sichtfeld der externen Kamera und Erfassen der zumindest drei Lagen des Roboterflanschs, insbesondere der Lagen des Trackers, durch die externe Kamera sowie gleichzeitiges Erfassen einer Transformation zwischen der Roboterbasis und dem Roboterflansch; und Durchführen, auf Basis der zumindest drei erfassten Lagen und der zumindest drei Transformationen, einer Hand-Augen-Kalibrierung mit Bestimmung einer Transformation von dem Roboterflansch zu der Roboterkamera (eye-in-hand) und zu der externen Kamera (eye-on-base).

Der Begriff Kalibrierungsmuster definiert ein in diesem Bereich der Bildanalyse verwendetes Muster. Insbesondere werden standardisierte Kalibrierungsmuster verwendet, wie etwa ein QR-Code oder ein schachbrettartiges Schwarz-Weiß-Muster mit zusätzlichen Markierungen.

Der Begriff "Position" meint eine geometrische Position im dreidimensionalen Raum, der insbesondere mittels Koordinaten eines kartesischen Koordinatensystems angegeben wird. Insbesondere kann die Position durch die drei Koordinaten X, Y und Z angegeben werden.

Der Begriff "Orientierung" wiederum gibt eine Ausrichtung (etwa an der Position) im Raum an. Man kann auch sagen, dass durch die Orientierung eine Ausrichtung angegeben wird mit Richtungs- bzw. Drehungsangabe im dreidimensionalen Raum. Insbesondere kann die Orientierung mittels drei Winkeln angegeben werden.

Der Begriff "Lage" umfasst sowohl eine Position als auch eine Orientierung. Insbesondere kann die Lage mittels sechs Koordinaten angegeben werden, drei Positionskoordinaten X, Y und Z sowie drei Winkelkoordinaten für die Orientierung.

Vorteilhafte Ausführungsformen sind in den Unteransprüchen beansprucht und werden insbesondere nachfolgend erläutert.

Gemäß einer Ausführungsform kann der Tracker an dem Roboterflansch angebracht sein und der Schritt Bewegen des Roboterflansches in die zumindest drei Lagen die Schritte aufweisen: Bestimmen einer Transformation zwischen dem Tracker und der externen Kamera in jeder Lage der zumindest drei Lagen, und Durchführen der Hand-Augen-Kalibrierung auf Basis der zumindest drei Transformationen von der Roboterbasis zu dem Roboterflansch und der zumindest drei Transformationen von dem Tracker zur externen Kamera mit Bestimmung einer Transformation zwischen dem Tracker und dem Roboterflansch. Ein am Roboterflansch angebrachter Tracker erlaubt eine besonders genaue Bestimmung seiner Lage durch die externe Kamera, insbesondere wenn diese externe Kamera als Tracking-Kamera ausgebildet ist.

Gemäß einer weiteren Ausführungsform des Kalibrierungsverfahrens kann das Kalibrierungsverfahren teiliterativ ausgeführt werden, wobei nach dem Schritt der Durchführung der Hand-Augen-Kalibrierung die Transformation zwischen dem Tracker und dem Roboterflansch, die Transformation zwischen der externen Kamera und dem Tacker sowie eine Vorwärtskinematik des Roboters angewandt wird, um drei neue Lagen des Roboterflansches zu bestimmen und den Roboterflansch mit dem Tracker (und der Roboterkamera) entsprechend in diese Lagen zu bewegen. Auf diese Weise kann die Präzision bzw. Genauigkeit der Hand-Augen-Kalibrierung nach einem ersten Durchlauf mit anfänglicher groben Kalibrierung, durch einen erneuten Durchlauf mit noch besser bestimmbaren Lagen bzw. Posen noch weiter erhöht werden.

Vorzugsweise kann der Schritt Bewegen der Roboterkamera: auf Basis einer ersten Transformation zwischen dem Roboterflansch und der Roboterkamera, insbesondere auf Basis einer hinterlegten groben/ geschätzten Transformation oder auf Basis eines 3D-Modells, insbesondere eines CAD-Modells, heuristisch und/oder systematisch durchgeführt werden, und/oder auf Basis von zufälligen Bewegungen durchgeführt werden, bis das optische Kalibrierungsmuster oder die externe Kamera detektiert wird. Im ersten Fall kann eine erste grobe Schätzung einer Transformation dafür dienen, nicht den gesamten Raum absuchen zu müssen, sondern schon systematisch eine Bewegung und Ausrichtung der Roboterkamera in die Richtung der externen Kamera, wenn bekannt, vorzunehmen. Anschließend kann in diesem begrenzten Bereich die externe Kamera gesucht werden bzw. das Kalibrierungsmuster. Dies senkt einen Rechenaufwand und eine benötigte Zeit, um die Lage der externen Kamera zu erfassen. Wenn das Kalibrierungsverfahren hingegen auf Basis von Zufallsbewegungen des Roboterarms eine Lage der externen Kamera detektiert, kann dies besonders robust und für neue Umgebungen eingesetzt werden, da keine Daten im Voraus bereitgestellt werden müssen.

Insbesondere kann der Schritt der Detektion des optischen Kalibrierungsmusters die Schritte aufweisen: Vergleichen von Teilbereichen der Aufnahme der Roboterkamera mit einem hinterlegten Kalibrierungsmuster und bei Erfassung mit Übereinstimmung des Teilbereiches mit dem hinterlegten Kalibrierungsmuster, Bestimmen der Lage des Kalibrierungsmusters mittels Bildanalyse und Bestimmen, auf Basis einer hinterlegten Transformation zwischen der Lage des Kalibrierungsmusters und der Lage der externen Kamera, einer Lage der externen Kamera. Es ist von Vorteil, wenn die externe Kamera selbst nicht direkt erfasst werden muss, da diese beispielsweise klein ist und eine Lage nicht so präzise bestimmbar ist, sondern indirekt über das Kalibrierungsmuster. Das Kalibrierungsmuster kann groß gestaltet und an der Basis entsprechend angeordnet werden, beispielsweise in einer solchen Höhe und Ausrichtung, dass die Roboterkamera eine besonders gute Sicht hat. Wenn das Kalibrierungsmuster ein planes Kalibrierungsmuster ist, so wird eine Bildanalyse für die Erfassung der Lage besonders einfach, im Gegensatz zu einer direkten Erfassung der Lage der externen Kamera.

Vorzugsweise kann der Schritt der Detektion einer Lage der externen Kamera die Schritte aufweisen: Vergleichen von Teilbereichen der Aufnahme der Roboterkamera, insbesondere von Teilstrukturen einer (dreidimensionalen) 3D-Aufnahme (etwa über eine Stereo-Kamera), mit einem hinterlegten geometrischen dreidimensionalen Modell, insbesondere einem CAD-Modell (alternativ oder zusätzlich kann auch eine 2D Kamera verwendet werden und die aufgenommenen 2D Bilder bzw. Aufnahmen durch Anwendung perspektivischer Ansichten mit dem 3D Modell verglichen werden), der externen Kamera und bei Detektion einer Übereinstimmung der erfassten geometrischen Struktur mit dem hinterlegten geometrischen Modell, Bestimmen einer Lage der externen Kamera durch Korrelieren der 3D-Strukturen. In dieser Ausgestaltung des Kalibrierungsverfahrens wird die externe Kamera durch die (Aufnahme der) Roboterkamera direkt erkannt und es wird ein geometrisches Fitting zwischen der erfassten externen Kamera und einem geometrischen Modell vorgenommen. Auf Basis dieser Anpassung des hinterlegten Modells (skalieren, drehen, verschieben und weitere) auf das erfasste Modell kann die Lage und das Sichtfeld der externen Kamera direkt ermittelt werden.

Gemäß einer Ausführungsform kann das Kalibrierungsverfahren ferner den Schritt einer geometrischen Kalibrierung aufweisen, insbesondere vor dem Schritt des Bewegens der Roboterkamera oder nach dem Schritt Durchführen der Hand-Augen-Kalibrierung. Da in der Medizintechnik eine besonders genaue Kalibrierung durchgeführt werden muss, um später Genauigkeiten einer Manipulation von wenigen Millimetern, insbesondere von unter einem Millimeter zu erhalten, wird die geometrische Kalibrierung durchgeführt, um die optischen Parameter der verwendeten Kamera zu bestimmen und bei einer späteren Bildanalyse auch eine optische Verzerrung, eine chromatische Aberration oder Ähnliches zu korrigieren. Insbesondere kann die geometrische Kamerakalibrierung mit der Hand-Auge-Kalibrierung während der Roboterbewegungen kombiniert werden. Insbesondere ist das Kalibrierungsmuster fixiert und bewegt sich im Raum nicht.

Insbesondere kann die geometrische Kalibrierung die Schritte aufweisen:
Bewegung der Roboterkamera (insbesondere systematisch oder auf der Grundlage einer Heuristik, die von einer groben Position des Kalibrierungsmusters relativ zur der Roboterkamera ausgeht, oder mit Hilfe von Zufallsbewegungen); Erfassen einer Szene durch die Roboterkamera und detektieren des Kalibrierungsmusters mit Hilfe von Bildverarbeitung und Objekterkennung. Sobald das Kalibrierungsmuster gefunden ist, ist die grobe Transformation zwischen der Roboterkamera und dem Kalibrierungsmuster bekannt. Unter Verwendung dieser Transformation und einer bekannten (groben) Transformation zwischen dem Auge in der Hand und dem Roboterflansch sowie der Vorwärtskinematik ist die Transformation zwischen dem Kalibrierungsmuster und der Roboterbasis bekannt. Insbesondere kann in einem nachfolgenden Schritt für eine geometrische Kalibrierung das Muster in verschiedenen Positionen im Fern- und Nahbereich der Kamera platziert werden und/oder die Kamera relativ zu dem Muster positioniert werden. Es muss auch in verschiedenen Positionen platziert werden, damit es in jeder Seite und Ecke sowie in der Mitte der Aufnahme der Roboterkamera (des Kamerabildes) erscheint. Außerdem muss insbesondere das Kalibrierungsmuster im Verhältnis zur Kamera schräg angestellt werden. Die Posen des Roboters (und damit der Eye-in-Hand-Kamera in Bezug auf das Kalibrierungsmuster) werden mit Hilfe der bekannten Transformationen berechnet, so dass das Muster an jeder der zuvor beschriebenen Positionen/Positionen der Aufnahme der Roboterkamera erscheint. Der Roboter, insbesondere der Roboterflansch mit der Roboterkamera, wird in jede dieser Stellungen bewegt, und die Roboterkamera nimmt Bilder des Kalibrierungsmusters in jeder dieser Stellungen auf. Schließlich wird die geometrische Kalibrierung anhand der aufgenommenen Bilder berechnet. Insbesondere kann eine grob bekannte Transformation zwischen der Roboterkamera und dem Roboterflansch erforderlich sein, welche aus einem CAD-Modell oder aus einer Hand-Auge-Kalibrierung stammt.

Gemäß einer weiteren Ausführungsform des Kalibrierungsverfahrens kann der Schritt Bewegen des Roboterflansches in zumindest drei unterschiedliche Lagen ferner den Schritt bzw. die Schritte aufweisen: Bestimmen eines besonders genau /präzise durch die externe Kamera erfassbaren Bereichs innerhalb des Sichtfelds der externen Kamera und Bewegen des Roboterflansches, insbesondere des Trackers, in diesen Bereich des Sichtfelds; und/oder Bestimmen einer Gelenkkonfiguration des Roboters, welche eine besonders genaue Erfassung der Lagen/ Posen erlaubt, und Bewegen in diese Lagen; und/oder Bewegen des Roboterflansches, insbesondere des Trackers, in zumindest drei im Sichtfeld der externen Kamera verteilte Lagen, insbesondere in solche Lagen, in denen ein Winkel zwischen dem Roboterflansch, insbesondere dem Tracker, und der externen Kamera zwischen klein und groß verteilt werden können. Die Lagen, in welche der Roboterflansch und insbesondere der Tracker gefahren werden sollen, werden also derart ausgewählt und bestimmt, dass eine besonders hohe Präzision einer Erfassung der Lage gewährleistet ist. Beispielsweise würden drei Lagen mit nur kleinen Abständen und Winkeländerungen zueinander nicht die geforderte Genauigkeit bereitstellen.

Vorzugsweise kann das Verfahren die Schritte aufweisen: Hand-Augen-Kalibrierung zwischen dem Roboter und der externen Kamera; und/oder Hand-Augen-Kalibrierung zwischen der Roboterkamera und der externen Kamera; und/oder Hand-Augen-Kalibrierung zwischen der Roboterkamera und dem Roboter, wobei in dem Fall, dass alle drei Hand-Augen-Kalibrierungen durchgeführt werden und somit redundante Transformationen vorliegen, eine Fehlerminimierung durchgeführt wird, insbesondere über eine Mittelwertabschätzung. Mit anderen Worten können also insbesondere die Kalibrierungen und ihre Varianten kombiniert werden, um einen Fehler zu minimieren (insbesondere mit Hilfe von Minimierungsverfahren). Dies bedeutet eine beliebige Kombination der folgenden Schritte: Durchführung einer Hand-Augen-Kalibrierung zwischen dem Roboter und der externen Kamera (eye-on-base); und/oder Durchführung einer Hand-Augen-Kalibrierung zwischen der externen Kamera (eye-on-base) und der Roboterkamera (eye-in-hand); und/oder Durchführung einer Hand-Augen-Kalibrierung zwischen der Roboterkamera (eye-in-hand) und dem Roboter. Insbesondere werden nach dem Sammeln der Proben die Kalibrierungen berechnet und der Fehler wird durch Optimierung der Berechnung der resultierenden Transformationen minimiert, so dass der Fehler minimal ist. Die kombinierten Kalibrierungsschritte können insbesondere auch nacheinander durchgeführt werden. Vorzugsweise kann eine oder es können mehrere Hand-Augen-Kalibrierungen gleichzeitig, insbesondere mit einer oder mehreren geometrischen Kalibrierungen durchgeführt werden. In diesem Fall nehmen die geometrisch zu kalibrierende(n) Kamera(s) für jede der ausgeführten Roboterposen bzw. Lagen des Roboterflansches, Aufnahmen (Bilder) des Kalibrierungsmusters auf, während die Transformationen von kalibrierten Systemen oder von Systemen, die nicht kalibriert werden müssen (beispielsweise einer Trackingkamera oder des Roboters mit präziser Kinematik), gesammelt werden. Die geometrischen Kalibrierungsschritte und die Hand-Auge-Kalibrierungsschritte können anschließend sequentiell oder parallel gelöst werden.

Insbesondere kann der Roboterflansch und/oder der an dem Roboterflansch angebrachte Tracker dafür verwendet werden, eine Kalibrierung des Roboters selbst durchzuführen. Die Erfassung kann über die externe Kamera als Trackingkamera erfolgen. Eine Ist-Lage des Trackers wird mit einer Soll-Lage des Trackers verglichen und bei einer Abweichung wir ein Korrekturfaktor für eine Anpassung an die Soll-Lage angewandt. Die Methoden des Kalibrierungsverfahrens können also insbesondere auch zur Kalibrierung des Roboters verwendet werden, indem gleichzeitig die Roboterposen erfasst werden, während die Kalibrierung zwischen der Roboterkamera und der externen Kamera durchgeführt wird. Die Roboterkinematik wird dann anhand der Daten der Nachverfolgung kalibriert und das Roboterkinematikmodell so optimiert, dass es zu den gesammelten Nachverfolgungsdaten vom Tracker des Roboterflanschs relativ zur der externen Kamera und/oder von der Roboterkamera relativ zu dem Kalibrierungsmuster passt. Vorzugsweise kann analog eine Tracking-Kamera alternativ oder zusätzlich zu dem Roboter kalibriert werden.

Insbesondere kann das Kalibrierungsverfahren die Schritte aufweisen: Anordnen einer statischen externen Kamera, Anordnen einer Kamera am Roboter als Roboterkamera, insbesondere im Sichtfeld der externen Kamera.

Hinsichtlich eines chirurgischen navigierten Assistenzsystem werden die Aufgaben dadurch gelöst, dass Dieses aufweist: zumindest einen Roboter, der einen an einer Roboterbasis angebundenen beweglichen Roboterarm mit einem Roboterflansch und insbesondere einen Tracker an dem Roboterflansch, aufweist, einer an den Roboterflansch angebundenen Roboterkamera (eye-in-hand), die mittels des Roboterarms bewegbar ist; und einem externen Kamerasystem mit zumindest einer externen Kamera, wobei der Roboterflansch, insbesondere der Tracker, und vorzugsweise die Roboterkamera, in ein Sichtfeld der externen Kamera bewegbar ist. Im Unterschied zum Stand der Technik weist das Assistenzsystem ferner eine Steuereinheit auf, die dafür vorgesehen und speziell angepasst ist:
die Roboterkamera mittels des ansteuerbaren Roboterarms zu bewegen und eine Aufnahme durch die Roboterkamera zu erstellen und zu verarbeiten;
in der erstellten Aufnahme ein optisches Kalibrierungsmuster mit vorbestimmter Transformation/ Relation zu der externen Kamera zu detektieren und/oder eine Lage der externen Kamera auf Basis der Aufnahme zu bestimmen;
auf Basis der ermittelten Lage der externen Kamera, eine Transformation zwischen der Roboterkamera und der externen Kamera sowie ein Sichtfeld der externen Kamera zu bestimmen;
den Roboterflansch, insbesondere den Tracker, in zumindest drei unterschiedliche Lagen im Sichtfeld der externen Kamera zu bewegen /zu steuern und durch die externe Kamera die zumindest drei Lagen des Roboterflanschs, insbesondere des Trackers, zu erfassen sowie gleichzeitig in jeder der zumindest drei Lagen eine Transformation zwischen der Roboterbasis und dem Roboterflansch zu erfassen; und
auf Basis der zumindest drei erfassten Lagen und der drei erfassten Transformationen, eine Hand-Augen-Kalibrierung durchzuführen, mit Bestimmung einer Transformation zwischen dem Roboterflansch und der Roboterkamera (eye-in-hand), insbesondere mit einer Transformation zwischen dem Tracker und der Roboterkamera, und einer Transformation zwischen dem Roboterflansch, insbesondere dem Tracker, und der externen Kamera (eye-on-base) und/oder einer Transformation zwischen Roboterflansch und dem Tracker.

Gemäß einer Ausführungsform kann die externe Kamera an einer Basis befestigt sein, wobei an der Basis zudem ein optisches Kalibrierungsmuster starr relativ zu der externen Kamera angeordnet ist und in einer Speichereinheit eine Transformation zwischen einer Lage des optischen Kalibrierungsmusters und der Lage der externen Kamera hinterlegt ist und der Steuereinheit bereitgestellt wird, um eine Kalibrierung durchzuführen. Die Steuereinheit erfasst über die Aufnahme der Roboterkamera in Verbindung mit einer Bildanalyse die Lage des optischen Kalibrierungsmusters und berechnet und bestimmt basierend auf dieser erfassten Lage sowie der bekannten und hinterlegten Transformation bzw. Transformationsmatrix zwischen der Lage des Kalibrierungsmusters und der externen Kamera schließlich die Lage der externen Kamera und deren Sichtfeld.

Vorzugsweise kann die externe Kamera eine Stereokamera für eine Nachverfolgung sein, insbesondere eine infrarotbasierte Stereokamera. Der Tracker kann insbesondere ein infrarotbasierter Tracker mit einer Vielzahl, insbesondere vier, voneinander beabstandeten Infrarotmarkern sein. Auf diese Weise lässt sich ein Tracker besonders gut räumlich erfassen.

Insbesondere kann der Roboterflansch, der Tracker und die Roboterkamera zueinander etwa einen gleichgroßen Abstand aufweisen. So sind diese drei Komponenten dicht bei-, jedoch gleich beabstandet zueinander angeordnet und mittels Bildanalyse kann die Steuereinheit eine zusätzliche Überprüfung der Lagen durchführen.

Gemäß einer Ausführungsform kann die Szene kann so eingerichtet werden, dass sich der Tracker auf dem Roboterflansch zunächst im Sichtfeld der externen Kamera befindet. Auf diese Weise wird eine Kalibrierung weiter unterstützt

Insbesondere kann das Kalibrierungsverfahren mit automatischer Hand-Augen-Kalibrierung ohne vorherige Kenntnis der Position der Roboterkamera relativ zum Flansch durchführen. Insbesondere kann, während sich der Tracker am Roboterflansch im Sichtfeld der externen Kamera befindet, der Roboter den Tracker in zufällige Lagen/ Posen bewegen. In jeder Lage wird eine Probe für die Kalibrierung des Hand-Augen-Systems gesammelt. Eine Probe weist eine Transformation von der Roboterbasis zu dem Roboterflansch und eine Transformation von der externen Kamera zum Tracker auf. Sobald mindestens drei Proben in den Lagen gesammelt wurden, in denen der Tracker von der externen Kamera erfasst wurde, kann eine (vorläufige) Hand-Augen-Kalibrierung berechnet werden, etwa über den Tsai-Lenz-Algorithmus. Danach ist die Transformation zwischen der Roboterbasis und der Roboterkamera. Insbesondere kann das Kalibrierungsverfahren fortgesetzt werden.

Insbesondere kann das für die geometrische Kalibrierung verwendete Kalibrierungsmuster dasselbe sein, wie das optische Kalibrierungsmuster zur Erfassung der Lage der externen Kamera. Auf diese Weise wird für das gesamte Kalibrierungsverfahren bzw. das Assistenzsystem nur ein einziges Kalibrierungsmuster benötigt.

Vorzugsweise kann das optische Kalibrierungsmuster auch auf einem Bildschirm angezeigt werden. So kann ein bereits im Operationssaal vorhandenes Gerät für die Kalibrierung benutzt werden und es muss kein eigenes Kalibrierungsmuster gedruckt und angebracht werden. Insbesondere kann als Kalibrierungsmuster, wenn dieses über einen Bildschirm, wie etwa einen OP-Monitor ausgegeben wird, ein zeitlich veränderliches, also ein dynamisches Kalibrierungsmuster sein. Beispielsweise kann sich eine Skalierung des Kalibrierungsmusters zeitlich verändern.

Insbesondere kann die Roboterkamera (eye-in-hand camera) auf die externe Kamera kalibriert bzw. registriert werden (mit Hilfe von Hand-Auge-Kalibrierungsalgorithmen). Hierbei muss die Transformation zwischen dem Kalibrierungsmuster und der externen Kamera konstant sein. Die Posen des Roboters werden so generiert, dass die externe Kamera in jeder Pose den Tracker auf dem Flansch erfasst (also im Sichtfeld ist) und im Gegenzug die Roboterkamera in jeder Lage/ Pose das Kalibrierungsmuster sieht und erfasst. In jeder Pose wird wieder eine Probe gesammelt. Eine Probe weist auf, insbesondere besteht aus, die Transformation zwischen dem Tracker am Roboterflansch und der externen Kamera sowie eine Transformation zwischen der Roboterkamera und dem Kalibrierungsmuster. Eine Durchführung einer entsprechend angepassten Hand-Augen-Kalibrierung liefert in diesem Fall die Transformationen zwischen dem Kalibrierungsmuster und der externen Kamera sowie die Transformation zwischen Roboterkamera und dem durch die externe Kamera getrackten bzw. nachverfolgten Tracker.

Gemäß einer weiteren Ausführungsform kann die externe Kamera an einer Basis befestigt sein und das optische Kalibrierungsmuster relativbeweglich zu der externen Kamera anorbar sein. Dabei verfolgt die externe Kamera das relativbewegliche Kalibrierungsmuster nach, insbesondere über einen starr an dem Kalibrierungsmuster angebrachten Kalibrierungstracker mit optischen Markern (etwa ein optischer Starrkörper-Tracker), wobei in diesem Fall in einer Speichereinheit eine statische Transformation (etwa eine Transformationsmatrix) von (der Lage des) dem Kalibrierungstracker zu (der Lage des) dem Kalibrierungsmuster hinterlegt ist, sodass die externe Kamera die Lage des Kalibrierungstrackers nachverfolgt und sich über die statische Transformation die Lage des Kalibrierungsmusters ermitteln lässt. Auch kann alternativ zu dem Kalibrierungstracker eine Methode der Bildverarbeitung eingesetzt werden, um das Kalibrierungsmuster nachzuverfolgen, sodass das Kalibrierungsmuster direkt erfasst und eine Lage bestimmt wird. Durch die Nachverfolgung des optischen Kalibrierungsmusters kann die die Steuereinheit basierend hierauf eine dynamische Transformation von der Lage des optischen Kalibrierungsmusters zu der Lage der externen Kamera berechnen und für eine Kalibrierung verwenden.

Gemäß einer Ausführungsform kann die externe Kamera eine optische Kamera sein. In diesem Fall kann eine geometrische Kalibrierung der externen Kamera durchgeführt werden. In diesem Fall bewegt der Roboter nicht (oder nicht nur) die Roboterkamera, sondern bewegt ein Kalibrierungsmuster (das in diesem Fall an dem Roboterflansch befestigt ist) vor der externen Kamera (eye-on-base), um dieses zu positionieren.

Vorzugsweise kann die Roboterkamera eine Tracking-Kamera sein.

Hinsichtlich eines computerlesbaren Speichermediums werden die Aufgaben dadurch gelöst, dass dieses Befehle umfasst, die bei einer Ausführung durch einen Computer diesen veranlassen, die Verfahrensschritte des Kalibrierungsverfahren gemäß der vorliegenden Offenbarung auszuführen.

Gemäß einer ganz bevorzugten Ausführungsform weist das Kalibierungsverfahren und eine speziell angepasste Steuereinheit des Assistenzsystems die nachstehenden Schritte bzw. Konfigurationen auf. Insbesondere kann die externe Kamera/ eye-on-base(-Kamera) eine Tracking-Kamera und die Roboterkamera eye-in-hand(-Kamera) eine optische 2D- oder 3D-Kamera sein, wobei ein Tracker starr an dem Roboterflansch und damit auch an der Roboterkamera angebracht ist. In einem ersten Schritt erfolgt eine Grobpositionierung des Roboters im Sichtfeld der externen Kamera (eye-on-base-Kamera). Der Roboter bewegt die Roboterkamera/ eye-in-hand im Raum (etwa systematisch, oder auf der Grundlage einer Heuristik, die eine grobe Position der externen Kamera/ eye-on-base relativ zur Roboterkamera/ eye-in-hand annimmt, oder mit Hilfe von Zufallsbewegungen). Hierbei ist eine grob bekannte Transformation zwischen dem Auge in der Hand und dem Roboterflansch erforderlich oder zumindest hilfreich. Diese kann z. B. aus einem CAD-Modell stammen. Die Roboterkamera erfasst die Szene und versucht die externe Kamera zu finden, indem sie entweder: einen Tracker oder ein bekanntes Muster (Kalibrierungsmuster) sucht, das im Bereich der externen Kamera angebracht ist, oder durch Bildverarbeitung und Identifizierung der externen Kamera in der Aufnahme (im Bild), wenn sie erfasst wurde diese direkt findet. Die Position, insbesondere die Lage, der externen Kamera wird bestimmt (insbesondere geschätzt) durch: Anhand der Position des Musters (Kalibrierungsmuster) und/oder des Trackers relativ zur externen Kamera (muss bekannt sein, kann aber sehr grob sein), oder durch Anpassung eines Kameramodells an die identifizierte Kamera und Ermittlung der groben Lage der externen Kamera. Mit Hilfe dieser Schritte ist eine (zumindest grobe) Transformation zwischen der externen Kamera und der Roboterkamera bekannt. Um fortzufahren, ist eine grobe Transformation zwischen der Roboterkamera und dem Roboterflansch hilfreich. Diese könnte beispielsweise von einem CAD-Modell stammen. Mit Hilfe dieser Transformation und der groben Transformation zwischen der Roboterkamera (eye-in-hand) und der externen Kamera (eye-on-base) in Kombination mit einem bekannten Sichtfeld der externen Kamera sind alle Informationen vorhanden, um die Roboterkamera durch den Roboter aktuiert in das Sichtfeld der externen Kamera zu bewegen. Hiernach folgt insbesondere der Schritt einer Lösung des Problems der Hand-Auge-Kalibrierung. Für diesen Schritt muss sich der Tracker an dem Roboterflansch nahe am Roboterflansch selbst befinden, damit angenommen werden kann, dass er sich bei einer Bewegung des Roboters ebenfalls im Sichtfeld der Eye-on-Base-Kamera befindet. Alternativ kann auch eine grobe Position des Trackers relativ zu dem Roboterflansch bekannt sein (etwa aus einem CAD-Modell). Für eine Hand-Augen-Kalibrierung wird der am Roboterflansch befestigte Tracker (zusammen mit dem Roboter) im Sichtfeld der externen Kamera bewegt. Das Sichtfeld der externen Kamera ist bekannt. Diese Informationen werden verwendet, um die Posen für den Roboter und damit die Lagen bzw. Posen für den Tracker zu berechnen. Die Lagen/ Posen des Trackers relativ zur externen Kamera werden so generiert, dass ein Fehler der Hand-Augen-Kalibrierung minimiert wird. Zur Berechnung der Posen können die folgenden Informationen berücksichtigt werden: Genauester Bereich innerhalb des Sichtfelds der externen Kamera; und/oder Gelenkkonfiguration des Roboters, bei der die beste Genauigkeit zu erwarten ist; und/oder Verteilung der Posen des Trackers innerhalb des Sichtfeldes der externen Kamera (eye-on-base); und/oder Verteilung der Posen des Trackers innerhalb des Sichtfeldes der externen Kamera, so dass die Winkel zwischen dem Tracker und der Kamera zwischen groß und klein verteilt werden können. Der Roboter wird zu den berechneten Posen gesteuert bzw. bewegt und es werden Proben sowohl für die Transformation zwischen dem Roboterflansch und der Roboterbasis als auch für die Transformation zwischen der externen Kamera und dem Tracker am Roboterflansch gesammelt. Schließlich wird Hand-Augen-Kalibrierung wird mit Hilfe bekannter Methoden, insbesondere mit dem Tsai-Lenz-Algorithmus, berechnet. Gemäß einem optionalen Schritt kann nach der Erfassung von mindestens drei Lagen/ Posen eine Zwischenkalibrierung der Hand-Augen-Kalibrierung mit bekannten Methoden wie dem Tsai-Lenz-Algorithmus berechnet werden. Diese neue Hand-Augen-Kalibrierung enthält eine Transformation zwischen dem Tracker und dem Roboterflansch. Unter Verwendung der Transformation von der externen Kamera zu dem Tracker, von dem Tracker zu dem Flansch und einer Vorwärtskinematik des Roboters können die Posen zwischen der externen Kamera und der Roboterbasis mit einer genaueren Transformation aktualisiert werden. Um weitere Stichproben für eine noch genauere Hand-Auge-Kalibrierung zu sammeln, werden die berechneten Posen mit der neuen Transformation neu berechnet, oder es werden neue Posen für den Roboter mit dem vorstehend beschriebenen Ansatz berechnet. Der Roboter fährt dann mit der Bewegung des Trackers fort, und es werden weitere Proben gesammelt.

Jegliche Offenbarung im Zusammenhang mit dem chirurgischen navigierten Assistenzsystem der vorliegenden Offenbarung gilt ebenso für das Kalibrierungsverfahren der vorliegenden Offenbarung sowie umgekehrt.

### Kurzbeschreibung der Figuren

Die vorliegende Offenbarung wird nachfolgend anhand bevorzugter Ausführungsformen unter Bezugnahme der begleitenden Figuren näher erläutert. Es zeigen:
- Fig. 1: eine schematische perspektivische Ansicht eines chirurgischen Assistenzsystems einer bevorzugten Ausführungsform, bei dem ein Kalibrierungsverfahren gemäß einer bevorzugten Ausführungsform zur automatischen Kalibrierung eingesetzt wird;
- Fig. 2: eine schematische perspektivische Ansicht eines chirurgischen Assistenzsystems gemäß einer weiteren bevorzugten Ausführungsform mit beweglichem Kalibrierungsmuster, bei dem ein Kalibrierungsverfahren gemäß einer bevorzugten Ausführungsform zur automatischen Kalibrierung eingesetzt wird; und
- Fig. 3: ein Flussdiagramm eines Kalibrierungsverfahrens gemäß einer weiteren bevorzugten Ausführungsform.

Die Figuren sind schematischer Natur und sollen nur dem Verständnis der vorliegenden Offenbarung dienen. Gleiche Elemente sind mit denselben Bezugszeichen versehen. Die Merkmale der verschiedenen Ausführungsbeispiele können untereinander ausgetauscht werden.

### Detaillierte Beschreibung bevorzugter Ausführungsformen

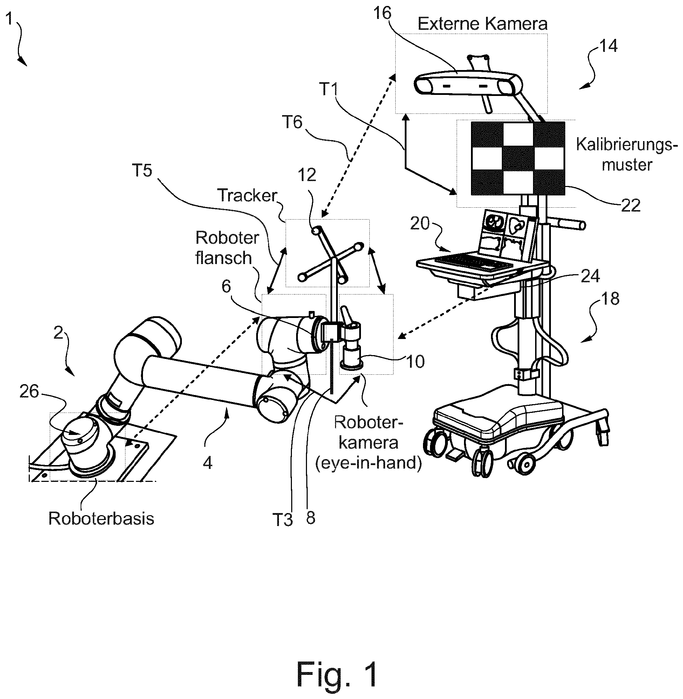

Fig. 1 zeigt ein chirurgisches Assistenzsystem 1 gemäß einer ersten bevorzugten Ausführungsform, welches eine automatisierte Kalibrierung, vorliegend ein Kalibrierungsverfahren gemäß einer ersten bevorzugten Ausführungsform durchführt.

Das chirurgische Assistenzsystem 1 hat einen Roboter 2 mit einem steuerbaren und beweglichen Roboterarm 4, der einen Endabschnitt mit einem Roboterflansch 6 aufweist. An diesem Roboterflansch 6 ist ein Endeffektor 8, etwa in Form einer Greifzange oder, wie vorliegend, eines Stabs, angebracht, um mit dem Endeffektor 8 ein Objekt bzw. einen Gegenstand zu manipulieren. Für eine Erfassung des Objekts und eine entsprechende Steuerung des Roboters ist am Roboterflansch 6 eine Kamera 10 am Roboter 2 (eye-in-hand; nachstehend Roboterkamera genannt) angebracht, deren Sichtfeld in die Richtung des Endeffektors 8 zeigt, um als Auge des Roboters 2 zu dienen und insbesondere einen Abschnitt des Endeffektors 8 optisch zu erfassen. Auf diese Weise können Objekte erfasst und nach Kalibrierung von Roboterkamera 10 zu Roboter 2 auch entsprechend angesteuert und manipuliert werden. Ebenso wie der Endeffektor 8 lässt sich auch die Roboterkamera 10 steuern und bewegen. Zudem ist an dem Roboterflansch 6 noch ein Tracker 12 in Form eines geometrischen Trackers mit vier zueinander beabstandeten Markierungspunkten vorgesehen, um mittels eines externen Kamerasystems 14 mit einer externen Kamera 16 den Tracker 12 und damit den Roboterflansch 6 besonders präzise räumlich hinsichtlich einer Lage, also einer Position und einer Orientierung, zu erfassen.

Die externe Kamera 16 an einer statischen Basis 18 ist auf den Roboter 2 gerichtet und erfasst, wenn der Roboterarm 4 mit dem Tracker 12 und der Roboterkamera 10 entsprechend seiner Kinematik in das Sichtfeld der externen Kamera 16 bewegt wird, den Roboterflansch 6, den Tracker 12 und die Roboterkamera 10.

Das chirurgische Assistenzsystem 1 weist für eine Funktion bzw. Konfiguration einer automatisierten Kalibrierung ferner eine Steuereinheit 20 auf die speziell dafür angepasst ist, eine automatische Kalibrierung zwischen der Roboterkamera 10, der externe Kamera 16 als auch dem Roboter 2 durchzuführen.

Im Unterschied zum Stand der Technik wird also nicht nur eine Kamera vorgesehen, sei es eine Kamera am Roboter oder eine externe Kamera, sondern es werden speziell zwei Kameras 10, 16 vorgesehen, nämlich eine steuerbare und aktiv führbare Roboterkamera 10 am Roboter 2 selbst als auch eine externe Kamera 16, die statisch an der Basis 18 befestigt ist und sich nicht mit dem Roboter 2 mitbewegt.

An der Basis 18 ist unterhalb der externen Kamera 16 eine plane Fläche mit einem aufgedrucktem optischen Kalibrierungsmuster 22 in Form eines Schachbrettmusters befestigt, das gegenüber der externen Kamera 16 eine definierte Lage aufweist. Alternativ kann auch über einen Monitor das optische Kalibrierungsmuster angezeigt werden. Insbesondere weist das Schachbrettmuster einzelne Vierecke mit weiteren Markierungen auf. Dieses optische Kalibrierungsmuster 22 dient der besonders einfachen Detektion einer Lage.

Die Steuereinheit 20 ist nun speziell dafür angepasst, in einem ersten Schritt, insbesondere nach einer Grobpositionierung des Roboters 2 in einem Sichtfeld der externen Kamera 16 (eye-on-base(-Kamera)), die an dem Roboterflansch 6 befestigte Roboterkamera 10 mit Hilfe von Zufallsbewegungen zufällig im Raum zu bewegen. Die Roboterkamera 10 erfasst dabei kontinuierlich die Umgebung bzw. erstellt eine kontinuierliche (Video-)Aufnahme A und stellt diese der Steuereinheit 20 computerlesbar bereit. Die Steuereinheit 20 wiederum analysiert diese Aufnahme A, um die externe Kamera 16 und deren Lage zu detektieren.

Konkret ist die Steuereinheit 20 dafür angepasst, in der Aufnahme A der Roboterkamera 10 das auch in einer Speichereinheit 24 hinterlegte und der Steuereinheit 20 bereitgestellte optische Kalibrierungsmuster 22 zu detektieren. Das optische Kalibrierungsmuster 22 ist besonderes einfach zu detektieren, da es in beliebiger, jedoch vorbestimmter, Relation zu der externen Kamera 16 angeordnet werden kann. Beispielsweise kann das optische Kalibrierungsmuster oberhalb von 1,5m angeordnet sein, so dass dieses durch medizinisches Fachpersonal oder hüfthoch angeordnete Objekte nicht gänzlich verdeckt wird. Auch ist dieses eine plane Fläche.

Auf Basis des durch die Roboterkamera 10 erfassten optischen Kalibrierungsmusters 22 bestimmt die Steuereinheit 20 dann eine Lage dieses optischen Kalibrierungsmusters 22. Da das Kalibrierungsmuster 22 abhängig von einem Winkel einer Normalen der planen Oberfläche zu einer direkten Verbindungslinie zwischen der Roboterkamera 10 in der Aufnahme A verzerrt aber rückberechenbar dargestellt ist, kann mittels üblichen Methoden der Bildanalyse eine Lage, also eine Position und eine Orientierung, bestimmt werden.

In der Speichereinheit 24 ist neben dem optischen Kalibrierungsmuster 22 auch eine erste Transformation, hier eine erste Transformationsmatrix T1, zwischen der Lage des Kalibrierungsmusters 22 und der Lage der externen Kamera 16 gespeichert. Man kann auch sagen, dass eine erste Transformationsmatrix T1 zwischen einem lokalen Koordinatensystem (KOS) des Kalibrierungsmusters 22 und dem lokalen KOS der externen Kamera 16 hinterlegt ist. Die Steuereinheit 20 bestimmt nun auf Basis der erfassten Lage des Kalibrierungsmusters 22 in Kombination mit der hinterlegten ersten Transformation T1 die Lage der externen Kamera 16 und kann so eine (grobe) zweite Transformation T2 zwischen der externen Kamera 16 und der Roboterkamera 10 berechnen, die noch weiter präzisiert werden kann. Die einzelnen lokalen Koordinatensysteme bzw. Gruppen sind für ein besseres Verständnis der vorliegenden Offenbarung schematisch als gestrichelte Boxen in Fig. 1 dargestellt.

Mit anderen Worten ist damit eine zweite Transformation T2 zwischen der Auge-an-Basis-Kamera 16 und der Auge-an-Hand-Kamera 10, sowie ein Sichtfeld der externen Kamera 16 bekannt. Um in der Kalibrierung fortzufahren, ist eine grobe dritte Transformation T3 zwischen der Roboterkamera 10 (Auge-in-Hand-Kamera) und dem Roboterflansch 6 hilfreich. In der Speichereinheit 24 ist dafür ein grobes 3D-Modell (CAD-Modell) hinterlegt, aus welcher die Steuereinheit 20 eine erste grobe Abschätzung einer solchen dritten Transformation T3 bestimmt.

Auf Basis der bestimmten zweiten Transformation T2 zwischen der externen Kamera 16 und der Roboterkamera 10 sowie optional der ersten groben dritten Transformation T3 zwischen dem Roboterflansch 6 und der Roboterkamera 10 sowie einem bekannten Sichtfeld der externen Kamera 16 sind alle notwendigen Daten vorhanden, um die Roboterkamera 10 in dem Sichtfeld der externen Kamera 16 zu bewegen und eine Kalibrierung durchzuführen.

Mit anderen Worten kann mit der zweiten Transformation T2 zwischen der Eye-in-Hand-Kamera 10 und der Eye-on-Base-Kamera 16 in Kombination mit einem bekannten Sichtfeld der Eye-on-Base-Kamera 16, die Eye-in-Hand-Kamera 10 robotermäßig in dem Sichtfeld der Eye-on-Base-Kamera 16 bewegt werden. Damit ist sozusagen ein optischer Rahmen für die Bewegung der Roboterkamera 10 innerhalb dieses festgelegten optischen Rahmens definiert.

In einem nächsten Schritt folgt die Lösung des Problems der Hand-Auge-Kalibrierung. Für diesen Schritt befindet sich der Tracker 12 nahe des Roboterflanschs 6 des Roboters 2, damit angenommen werden kann, dass er sich, vorzugsweise neben der Roboterkamera 10 und vorzugsweise dem Roboterflansch 6, ebenfalls im Sichtfeld der Roboterkamera 10 befindet. Alternativ kann auch eine grobe Lage des Trackers 12 relativ zum Roboterflansch 6 bekannt sein, insbesondere aus einem in der Speichereinheit 24 hinterlegten CAD-Modell.

Für eine Hand-Augen-Kalibrierung (hand-eye-calibration) wird der am Roboterflansch 6 des Roboters 2 befestigte Tracker 12 mittels der Steuereinheit 20 zusammen mit dem Roboterarm 4 in dem bekannten Sichtfeld der externen Kamera 16 bewegt.

Es werden Daten verwendet, um die Posen für den Roboter 2 und damit für den Tracker 12 zu berechnen. Die Posen des Trackers 12 relativ zur externen Kamera 16 werden so generiert, dass ein Hand-Augen-Kalibrierungsfehler minimiert wird. Zur Berechnung der Posen werden insbesondere die folgenden Daten berücksichtigt: ein genauester Bereich innerhalb des Sichtfelds der externen Kamera 16; eine solche Gelenkkonfiguration des Roboters 2, bei der die beste Genauigkeit zu erwarten ist; eine Verteilung der Lagen/ Posen des Trackers 12 innerhalb des gesamten Sichtfeldes der externen Kamera 16, um möglichst unterschiedliche Posen zu erfassen; und/oder Verteilung der Lagen/ Posen des Trackers 12 innerhalb des Sichtfeldes der externen Kamera 16, so dass Winkel zwischen dem Tracker 12 und der externen Kamera 16 zwischen Groß und Klein unterschiedlich gewählt und angesteuert werden können. Die Steuereinheit 20 bestimmt dabei zumindest drei Lagen/ Posen für den Roboterflansch 6 mit dem Tracker 12.

Anschließend steuert die Steuereinheit 20 den Roboter 2 derart, dass dieser in die berechneten Lagen bewegt wird. Es werden dabei Daten (Proben) sowohl für eine Transformation zwischen dem Roboterflansch 6 und einer Roboterbasis 26 als auch für eine sechste Transformation T6 zwischen der externen Kamera 16 und dem Tracker 12 am Flansch gesammelt.

Vorzugsweise kann in einem optionalen Schritt, nach der Erfassung von zumindest drei Posen, eine Zwischenkalibrierung der Hand-Augen-Kalibrierung (hand-eye-calibration) mit bekannten Methoden, insbesondere dem Tsai-Lenz-Algorithmus, berechnet werden. Die neue Hand-Augen-Kalibrierung enthält eine Transformation zwischen dem Tracker 12 und dem Roboterflansch 6. Unter Verwendung der sechsten Transformation T6 von der externen Kamera 16 zum Tracker 12, einer fünften Transformation T5 vom Tracker 12 zum Roboterflansch 6 und einer Vorwärtskinematik des Roboters 2 kann eine bekannte vierte Transformation T4 und/oder Pose zwischen der externen Kamera 16 und der Roboterbasis 26 mit einer genaueren vierten Transformation T4 aktualisiert werden. Um weitere (Stich)Proben für eine noch genauere Hand-Augen-Kalibrierung zu sammeln, werden die zuvor berechneten Posen mit der nun neuen Transformation neu berechnet. Alternativ oder zusätzlich können auch neue Posen für den Roboter 2 mit dem vorstehenden Ansatz berechnet werden. Der Roboter 2 fährt dann mit der Bewegung des Trackers 12 fort, und es werden entsprechende Stichproben gesammelt.

Schließlich berechnet die Steuereinheit 20 dann die Hand-Augen-Kalibrierung mit Hilfe bekannter Methoden, insbesondere dem Tsai-Lenz-Algorithmus, auf Basis der erfassten Proben. Damit berechnet die Steuereinheit 20 insbesondere die dritte Transformation T3 zwischen der Roboterkamera 10 und dem Roboterflansch 6 und liefert eine Kalibrierung bzw. Registrierung zwischen der Roboterkamera 10 und dem Roboter 2 sowie auch eine Kalibrierung zwischen der externen Kamera 16 und/oder dem Tracker 12 und dem Roboterflansch 6 und damit des Roboters 2. Damit ist das Problem der Hand-Augen-Kalibrierung gelöst.

In Fig. 1 sind zwischen dem Roboter 2 und den zwei Kameras 10, 16 alle relevanten Systemnamen und Transformationen abgebildet. Die durchgezogenen Pfeile stellen Transformationen dar, die aus der Hand-Auge-Kalibrierung (hand-eye-calibration) berechnet werden können. Die gestrichelten Pfeile stehen für Transformationen, die gemessen werden können und insbesondere kalibriert werden können, vorzugsweise über eine geometrische Kalibrierung.

Zusätzlich zu der vorstehend beschriebenen Konfiguration wird die Roboterkamera 10, als optionale Kalibrierung, auch noch geometrisch kalibriert. Diese geometrische Kalibrierung kann die Steuereinheit 20 optional durchführen. Diese geometrische Kalibrierung ist nur erforderlich, wenn die Roboterkamera 10 und/oder die externe Kamera auch geometrisch kalibriert werden soll. Die geometrische Kalibrierung ist unabhängig von der vorstehenden Hand-Auge-Kalibrierung.

Für diesen Schritt wird ein Kamerakalibrierungsalgorithmus verwendet. Für die geometrische Kamerakalibrierung wird abermals ein Kalibrierungsmuster benötigt. Diese Kalibrierungsmuster kann insbesondere das optische Kalibrierungsmuster 22 sein. Für die geometrische Kalibrierung muss das Kalibrierungsmuster im Sichtfeld der Kamera platziert werden, und die Kalibrierung erfolgt unter Zuhilfenahme der bekannten Geometrie des Kalibrierungsmusters und eines entsprechenden Kalibrierungsalgorithmus, um (optische) Kameraparameter wie Brennweite und Verzeichnungskoeffizienten aus den erfassten Verzerrungen zu errechnen. Entscheidend ist, dass das Kalibrierungsmuster relativ zu der Kamera bewegt wird, entweder durch Verschieben des Kalibrierungsmusters oder durch Bewegen der Kamera.

Im Unterschied zum Stand der Technik wird jedoch in der vorliegenden Offenbarung die geometrische Kamerakalibrierung mit der Hand-Auge-Kalibrierung während der Bewegung des Roboters kombiniert. In diesem Szenario ist das Kalibrierungsmuster an der Basis fixiert.

Dabei ist die Steuereinheit 20 dafür angepasst, die nachstehenden Teilschritte durchzuführen. In einem ersten Schritt wird die Roboterkamera 10 wieder mittels des Roboterarms 4 bewegt, etwa systematisch, auf der Grundlage einer Heuristik, die von einer groben Position des Kalibrierungsmusters relativ zu der Roboterkamera ausgeht (mittels einer bekannten groben dritten Transformation zwischen der Roboterkamera 10 und dem Roboterflansch 6), oder wieder mit Hilfe von Zufallsbewegungen.

Die Roboterkamera 10 erfasst wieder die Umgebung und ist dafür angepasst, das Kalibrierungsmuster 22 mit Hilfe von Bildverarbeitung und Objekterkennung zu detektieren. Sobald das (geometrische) Kalibrierungsmuster 22 detektiert wurde, ist die grobe Transformation zwischen dem Roboterkamera 10 und dem (geometrischen) Kalibrierungsmuster 22 bekannt. Unter Verwendung dieser Transformation und einer bekannten (groben) dritten Transformation T3 zwischen der Roboterkamera 10 und dem Roboterflansch 6 sowie der Vorwärtskinematik ist die Transformation zwischen dem Kalibrierungsmuster 22 und der Roboterbasis 26 durch die Steuereinheit 20 bestimmbar.

Die Steuereinheit 20 ist dafür angepasst, in einem darauffolgenden Schritt für eine Kalibrierung das Kalibrierungsmuster 22 in verschiedenen Positionen im Fern- und Nahbereich der Roboterkamera 10 zu platzieren. Zudem ist die Steuereinheit 20 dafür angepasst das Kalibrierungsmuster 22 auch in verschiedenen Positionen zu platzieren, damit es auf jeder Seite und in jeder Ecke sowie in der Mitte des Kamerabildes der Roboterkamera 10 erscheint. Außerdem ist die Steuereinheit 20 dafür angepasst, das Kalibrierungsmuster 22 im Verhältnis zur Roboterkamera 10 schräg anzustellen. Bei einem traditionellen Ansatz müssen diese Schritte manuell durchgeführt werden. Gemäß der vorliegenden Offenbarung werden die Posen des Roboters 2 (und damit der Roboterkamera 10 in Bezug auf das Kalibrierungsmuster 22) mit Hilfe der bekannten Transformationen berechnet, so dass das Kalibrierungsmuster 22 an jeder der zuvor beschriebenen Positionen des durch die Roboterkamera 10 aufgenommenen Aufnahme A erscheint und durch die Steuereinheit 20 entsprechend verarbeitet werden kann, um die geometrische Kalibrierung durchzuführen. Der Roboter 2 wird, gesteuert durch die Steuereinheit 20, in jede dieser Stellungen bewegt, und die Roboterkamera 10 nimmt Aufnahmen A (Bilder) des Kalibrierungsmusters 22 in jeder dieser Stellungen auf. Danach berechnet die Steuereinheit 20 anhand der aufgenommenen Bilder die geometrische Kalibrierung der Roboterkamera 10.

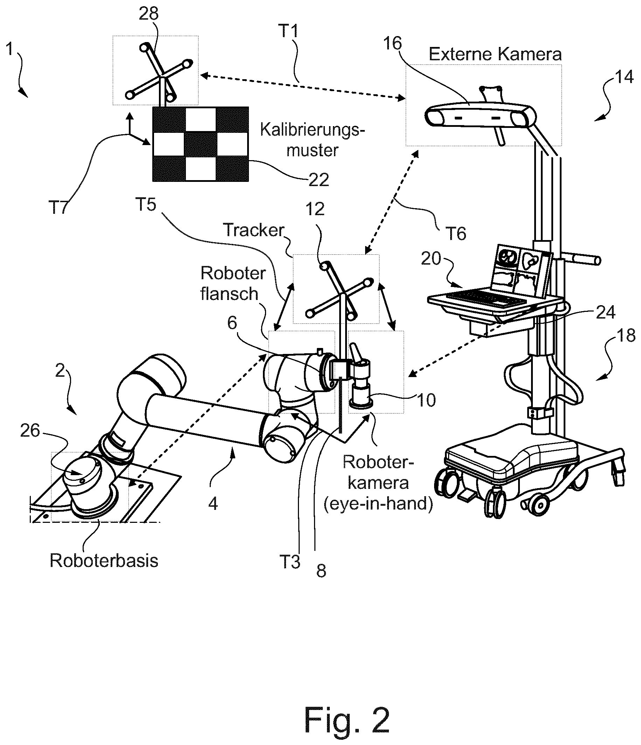

Fig. 2 zeigt eine schematische perspektivische Ansicht eines chirurgischen Assistenzsystems 1 gemäß einer weiteren bevorzugten Ausführungsform. Im Unterschied zu der Ausführungsform aus Fig. 1 weist das chirurgische Assistenzsystem 1 dieser Ausführungsform ein zu der externen Kamera 16 relativbewegliches Kalibrierungsmuster 22 auf, welches an verschiedenen Positionen im Raum angeordnet werden kann, beispielsweise manuell gehalten oder über einen Arm, der etwa aktiv aktuierbar ist. Dabei ist das relativbewegliche Kalibrierungsmuster 22 im Sichtfeld der externen Kamera 16 angeordnet und die externe Kamera 16 erfasst eine Lage des Kalibrierungsmusters 22 zu der externen Kamera 16 über einen fest an dem Kalibrierungsmuster 22 angebrachten Kalibrierungstracker 28 mit optischen Markern, wobei der Steuereinheit 20 eine statische siebte Transformation T7 von dem Kalibrierungstracker 28 zu dem Kalibrierungsmuster 22 bereitgestellt wird, etwa als numerische Matrix in einer Speichereinheit hinterlegt ist. Dadurch, dass das Kalibrierungsmuster 22 in dieser Ausführungsform nicht starr angebracht, sondern relativbeweglich ist, kann dieses bei einer Operation an günstigen Orten im Operationssaal angeordnet werden, an denen das Kalibrierungsmuster 22 nicht stört oder an denen es eine besonders gute Position für eine genaue Kalibrierung bietet. Die externe Kamera 16 erfasst also eine (dynamische) Lage des Kalibrierungsmusters 22 und stellt eine (dynamische) Transformation, etwa als Transformationsmatrix, für eine Kalibrierung bereit. Wird also das Kalibrierungsmuster 22 erfasst, so kann über die ermittelte dynamische Transformation auch die Lage der externen Kamera 16 und die zweite Transformation T2 zwischen der Roboterkamera 10 und der externen Kamera 16 bestimmt werden.

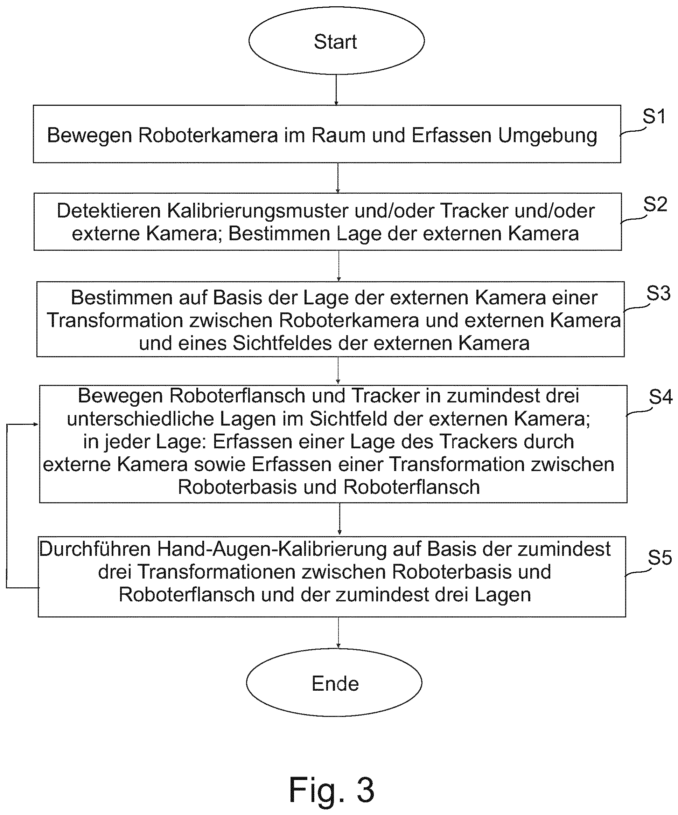

Fig. 3 zeigt in Form eines Flussdiagramms einen Verfahrensablauf eines Kalibrierungsverfahrens gemäß einer bevorzugten Ausführungsform. Dieses kann insbesondere bei einem chirurgischen navigierten Assistenzsystem aus Fig. 1 eingesetzt werden.

In einem ersten Schritt S1 wird eine Roboterkamera, als eine Kamera, welche an einem Roboterarm angebunden und über diesen bewegt werden kann (eye-in-hand), im Raum bewegt und es wird eine kontinuierliche Aufnahme durch die Roboterkamera erstellt und damit eine Szene bzw. Umgebung erfasst.

In einem Schritt S2 wird ein vorbestimmtes optisches Kalibrierungsmuster und/oder ein externer Tracker und dessen Lage mit vorbestimmter Transformation zu einer Lage der externen Kamera und/oder eine Lage der externen Kamera auf Basis der Aufnahme detektiert.

In einem Schritt S3 wird dann, auf Basis der ermittelten Lage der externen Kamera, einer Transformation zwischen der Roboterkamera und der externen Kamera und ein Sichtfeld der externen Kamera bestimmt.

In einem Schritt S4 erfolgt ein Bewegen des Roboterflansches mit einem am Roboterflansch angebrachten Tracker, in zumindest drei unterschiedliche Lagen in dem Sichtfeld der externen Kamera und Erfassen der zumindest drei Lagen des Trackers, durch die externe Kamera sowie gleichzeitiges Erfassen einer Transformation zwischen der Roboterbasis und dem Roboterflansch.

Schließlich erfolgt in Schritt S5 auf Basis der zumindest drei erfassten Lagen und der zumindest drei Transformationen zwischen der Roboterbasis und dem Roboterflansch, eine Hand-Augen-Kalibrierung mit Bestimmung einer Transformation zu der externen Kamera.

### Bezugszeichenliste

- 1: Chirurgisches Assistenzsystem
- 2: Roboter
- 4: Roboterarm
- 6: Roboterflansch
- 8: Endeffektor
- 10: Roboterkamera
- 12: Tracker
- 14: externes Kamerasystem
- 16: externe Kamera
- 18: Basis
- 20: Steuereinheit
- 22: Kalibrierungsmuster
- 24: Speichereinheit
- 26: Roboterbasis
- 28: Kalibrierungstracker
- A: Aufnahme Roboterkamera

- S1: Schritt Bewegen Roboterkamera
- S2: Schritt Detektion Lage der externen Kamera
- S3: Schritt Bestimmen Transformation und Sichtfeld der externen Kamera
- S4: Schritt Bewegen Tracker am Roboterflansch in mehrere Lagen in dem Sichtfeld
- S5: Durchführung der Hand-Auge-Kalibrierung

- T1: erste Transformation
- T2: zweite Transformation
- T3: dritte Transformation
- T4: vierte Transformation
- T5: fünfte Transformation
- T6: sechste Transformation
- T7: siebte Transformation

## Patentansprüche

1. Kalibrierungsverfahren zur automatisierten Kalibrierung einer Roboterkamera (10) zu einem medizinischen, insbesondere chirurgischen, Roboter (2), und zur automatisierten Kalibrierung eines externen Kamerasystems mit zumindest einer externen Kamera (16) zu dem medizinischen Roboter (2), wobei die Roboterkamera (10) an einem Roboterflansch (6) eines Roboterarms (4), der an eine Roboterbasis (26) angebunden ist, befestigt ist, umfassend die Schritte:
(a) Bewegen der Roboterkamera (10) mittels des Roboterarms (4) unter Aufnahme (A) durch die Roboterkamera (10);
(b) Detektion, auf Basis der Aufnahme (A), einer Lage eines vorbestimmten optischen Kalibrierungsmusters (22) und/ oder eines fest an dem Kalibrierungsmuster (22) angebrachten Kalibrierungstrackers (28), jeweils mit vorbestimmter Transformation von dieser detektierten Lage zu einer Lage der externen Kamera (16), und/ oder Detektion, auf Basis der Aufnahme (A), einer Lage der externen Kamera (16), wobei eine vorbestimmte erste Transformation (T1) die Lage des vorbestimmten optischen Kalibrierungsmusters (22) und/ oder des Kalibrierungstrackers (28) zu einer Lage der externen Kamera (16) abbildet;
(c) Bestimmen, auf Basis der ermittelten Lage der externen Kamera (16), einer zweiten Transformation (T2) zwischen der Roboterkamera (10) und der externen Kamera (16) und Bestimmen eines Sichtfelds der externen Kamera (16);
(d) Bewegen des Roboterflansches (6) in zumindest drei unterschiedliche Lagen im Sichtfeld der externen Kamera (16);
(e) Erfassen der zumindest drei Lagen des Roboterflanschs (6) durch die externe Kamera (16) ;
(f) Bestimmen einer dritten Transformation (T3) von dem Roboterflansch (6) zu der Roboterkamera (10) und/ oder einer vierten Transformation (T4) von der externen Kamera (16) zu der Roboterbasis (26); und
(g) Durchführen, auf Basis der zumindest drei erfassten Lagen des Roboterflanschs (6) und zumindest drei der bestimmten ersten, zweiten, dritten oder vierten Transformationen, einer Hand-Augen-Kalibrierung.

2. Kalibrierungsverfahren nach Anspruch 1, **dadurch gekennzeichnet, dass** ein Tracker (12) an dem Roboterflansch (6) befestigt ist,
dass zumindest drei Lagen des Trackers (12) durch die externe Kamera (16) erfasst werden,
dass eine fünfte Transformation (T5) von dem Roboterflansch (6) zu dem Tracker (12) bekannt ist oder, auf Basis der zumindest drei erfassten Lagen des Roboterflanschs (6) und des am Roboterflansch (6) befestigten Trackers (12), bestimmt wird,
und dass eine Hand-Augen-Kalibrierung, auf Basis der zumindest drei erfassten Lagen des Roboterflanschs (6) und zumindest drei der bestimmten ersten, zweiten, dritten oder vierten Transformationen oder bekannten oder bestimmten fünften Transformation, durchgeführt wird.

3. Kalibrierungsverfahren nach Anspruch 2, **dadurch gekennzeichnet, dass** die Schritte Bewegen des Roboterflansches und Durchführen der Hand-Augen-Kalibrierung des Kalibrierungsverfahrens iterativ ausgeführt werden und nach einem ersten Durchgang der Durchführung der Hand-Augen-Kalibrierung die bekannte oder ermittelte fünfte Transformation (T5) zwischen dem Tracker (12) und dem Roboterflansch (6), eine sechste Transformation (T6) zwischen der externen Kamera (16) und dem Tracker (12) sowie eine Vorwärtskinematik des Roboters (2) angewandt wird, um neue Lagen des Roboterflansches (6) zu bestimmen und den Roboterflansch (6) entsprechend in diese Lagen für die nächste Iteration zu bewegen.

4. Kalibrierungsverfahren nach einem der vorstehenden Ansprüche, **dadurch gekennzeichnet, dass** der Schritt Bewegen der Roboterkamera (10) auf Basis von zufälligen Bewegungen durchgeführt wird, bis das optische Kalibrierungsmuster (22) oder der externe Tracker oder die externe Kamera (16) detektiert wird.

5. Kalibrierungsverfahren nach einem der vorstehenden Ansprüche, **dadurch gekennzeichnet, dass** der Schritt der Detektion des optischen Kalibrierungsmusters (22) die Schritte aufweist:
Vergleichen von Teilbereichen der Aufnahme (A) der Roboterkamera (10) mit einem hinterlegten Kalibrierungsmuster (22) und bei Übereinstimmung: Bestimmen der Lage des Kalibrierungsmusters (22) mittels Bildanalyse und Bestimmen, auf Basis einer hinterlegten ersten Transformation (T1) zwischen der Lage des Kalibrierungsmusters (22) und der Lage der externen Kamera (16), einer Lage der externen Kamera (16).

6. Kalibrierungsverfahren nach einem der vorstehenden Ansprüche, **dadurch gekennzeichnet, dass** der Schritt der Detektion einer Lage der externen Kamera (16) die Schritte aufweist:
Vergleichen von Teilbereichen der Aufnahme (A) der Roboterkamera (10), insbesondere einer 3D-Aufnahme, mit einem hinterlegten geometrischen Modell, insbesondere einem CAD-Modell, der externen Kamera (16) und bei Detektion einer Übereinstimmung mit dem hinterlegten geometrischen Modell, Bestimmen einer Lage der externen Kamera (16) durch Korrelieren der dreidimensionalen Strukturen.

7. Kalibrierungsverfahren nach einem der vorstehenden Ansprüche, **dadurch gekennzeichnet, dass** das Kalibrierungsverfahren ferner den Schritt einer geometrischen Kalibrierung aufweist, insbesondere vor dem Schritt des Bewegens der Roboterkamera (10) oder nach dem Schritt der Durchführung der Hand-Augen-Kalibrierung.

8. Chirurgisches navigiertes Assistenzsystem (1) mit
zumindest einem Roboter (2), der einen an einer Roboterbasis (26) angebundenen beweglichen Roboterarm (4) mit einem Roboterflansch (6) und insbesondere einen Tracker (12) an dem Roboterflansch (6), aufweist;
einer an den Roboterflansch (6) angebundenen Roboterkamera (10), die mittels des Roboterarms (4) bewegbar ist; und
einem externen Kalibrierungsmuster (22), sowie einem, vorzugsweise am Kalibrierungsmuster (2) fest angebrachten, Kalibrierungstracker (28),
einem externen Kamerasystem (14) mit zumindest einer externen Kamera (16), wobei der Roboterflansch (6), insbesondere der Tracker (12) und/oder die Roboterkamera (10), in ein Sichtfeld der externen Kamera (16) bewegbar ist, **dadurch gekennzeichnet, dass** das Assistenzsystem (1) ferner eine Steuereinheit (20) aufweist, die dafür angepasst ist, das Kalibrierungsverfahren nach einem der vorstehenden Ansprüche 1 bis 7 durchzuführen.

9. Chirurgisches Assistenzsystem (1) nach Anspruchs 8, **dadurch gekennzeichnet, dass**
die externe Kamera (16) an einer Basis (18) befestigt ist und an der Basis (18) zudem das optische Kalibrierungsmuster (22) starr relativ zu der externen Kamera (16) angeordnet ist, und in einer Speichereinheit (24) eine statische erste Transformation (T1) zwischen einer Lage des optischen Kalibrierungsmusters (22) und der Lage der externen Kamera (16) hinterlegt ist und der Steuereinheit (20) für die Bestimmung der Lage der externen Kamera (16) bereitgestellt wird, oder
die externe Kamera (16) an einer Basis (18) befestigt ist und das optische Kalibrierungsmuster (22) relativbeweglich zu der externen Kamera (16) ist, welche das relativbewegliche Kalibrierungsmuster (22) nachverfolgt, insbesondere über den starr an dem Kalibrierungsmuster (22) angebrachten Kalibrierungstracker (28) mit optischen Markern und in einer Speichereinheit hinterlegter statischer siebter Transformation (T7) zu dem Kalibrierungsmuster (22), wobei die Steuereinheit (20) basierend hierauf eine dynamische erste Transformation (T1) von der Lage des optischen Kalibrierungsmusters (22) zu der Lage der externen Kamera (16) berechnet.

10. Chirurgisches Assistenzsystem (1) nach Anspruch 8 oder 9, **dadurch gekennzeichnet, dass** die externe Kamera (16) eine Stereokamera für eine Nachverfolgung ist, insbesondere eine infrarotbasierte Stereo-Trackingkamera, und der Tracker (12) am Roboterflansch (6) insbesondere ein infrarotbasierter Tracker mit einer Vielzahl von zueinander beabstandeten Infrarotmarkern ist.

11. Chirurgisches Assistenzsystem (1) nach einem der Ansprüche 8 bis 10, **dadurch gekennzeichnet, dass** der Roboterflansch (6), der Tracker (12) und die Roboterkamera (10) starr zueinander sind und insbesondere einen gleichen Abstand zueinander aufweisen.

12. Computerlesbares Speichermedium, das Befehle umfasst, die bei einer Ausführung durch einen Computer diesen veranlassen, die Verfahrensschritte des Kalibrierungsverfahren gemäß einem der Ansprüche 1 bis 7 auszuführen.

## Claims

1. A calibration method for automated calibration of a robot camera (10) in relation to a medical, in particular to a surgical robot (2), and for automated calibration of an external camera system that includes at least one external camera (16), in relation to the medical robot (2), the robot camera (10) being fastened on a robot flange (6) of a robot arm (4), which is connected to a robot base (26), comprising the steps of:
(a) moving the robot camera (10) by way of the robot arm (4) during capturing (A) by the robot camera (10);
(b) detecting, on the basis of the capturing (A), the position of a predefined optical calibration pattern (22) and/or of an external calibration tracker (28) firmly fixed on the calibration pattern (22), each having a predefined transformation from this detected position to a position of the external camera (16), and/or detecting, on the basis of the captured image (A), the position of the external camera (16), wherein a predefined first transformation (T1) images the position of the predefined optical calibration pattern (22) and/or of the calibration tracker (28) in relation to a position of the external camera (16);
(c) determining, on the basis of the ascertained position of the external camera (16), a second transformation (T2) between the robot camera (10) and the external camera (16), and determining a field of view of the external camera (16);
(d) moving the robot flange (6) into at least three different positions in the field of view of the external camera (16);
(e) sensing the at least three positions of the robot flange (6) by the external camera (16);
(f) determining a third transformation (T3) from the robot flange (6) to the robot camera (10) and/or a fourth transformation (T4) from the external camera (16) to the robot base (26); and
(g) carrying out, on the basis of the at least three sensed positions of the robot flange (6) and at least three of the determined first, second, third or fourth transformations, a hand-eye calibration.

2. The calibration method according to claim 1, **characterized in that** a tracker (12) is fastened on the robot flange (6),
that at least three positions of the tracker (12) are sensed by the external camera (16);
that a fifth transformation (T5) from the robot flange (6) to the tracker (12) is known or is determined, based on the at least three detected positions of the robot flange (6) and the tracker (12) fastened to the robot flange (6),
and that a hand-eye calibration is carried out on the basis of the at least three sensed positions of the robot flange (6) and at least three of the sensed first, second, third or fourth transformations or known or determined fifth transformation.

3. The calibration method according to claim 2, **characterized in that** the steps of moving the robot flange and carrying out the hand-eye calibration of the calibration method are performed iteratively, and that after a first round of carrying out the hand-eye calibration, the known or determined fifth transformation (T5) between the tracker (12) and the robot flange (6), a sixth transformation (T6) between the external camera (16) and the tracker (12), as well as forward kinematics of the robot (2) are used to determine new positions of the robot flange (6) and to correspondingly move the robot flange (6) into these positions for the next iteration.

4. The calibration method according to any of the preceding claims, **characterized in that** the step of moving the robot camera (10) is carried out on the basis of random movements until the optical calibration pattern (22) or the external tracker or the external camera (16) is detected.

5. The calibration method according to any of the preceding claims, **characterized in that** the step of detecting the optical calibration pattern (22) comprises the steps of:
comparing sections of the capturing (A) of the robot camera (10) with a stored calibration pattern and in case of conformity: determining the position of the calibration pattern (22) by means of image analysis and determining, on the basis of a stored first transformation (T1) between the position of the calibration pattern (22) and the position of the external camera (16), a position of the external camera (16).

6. The calibration method according to any of the preceding claims, **characterized in that** the step of detecting a position of the external camera (16) comprises the steps of:
comparing sections of the capturing (A) of the robot camera (10), in particular a 3D image, with a stored geometric model, more particularly a CAD model, of the external camera (16), and in the case of detecting a conformity with the stored geometric model, determining a position of the external camera (16) by correlating the three-dimensional structures.

7. The calibration method according to any of the preceding claims, **characterized in that** the calibration method further comprises the step of a geometric calibration, more particularly prior to the step of moving the robot camera (10) or after the step of carrying out the hand-eye calibration.

8. A surgical navigated assistance system (1), comprising at least one robot (2) including a movable robot arm (4) connected to a robot base (26) and including a robot flange (6), and more particularly a tracker (12) on the robot flange (6);
a robot camera (10) connected to the robot flange (6) and movable by means of the robot arm (4);
an external calibration pattern (22) as well as a calibration tracker (28) preferably firmly fixed to the calibration pattern (2),
an external camera system (14) comprising at least one external camera (16), wherein the robot flange (6), more particularly the tracker (12), and/or the robot camera (10) is movable into a field of view of the external camera (16), **characterized in that** the assistance system (1) further comprises a control unit (20) adapted to carry out the calibration method according to any of preceding claims 1 to 7.

9. The surgical assistance system (1) according to claim 8, **characterized in that**
the external camera (16) is fastened on a base (18) and, additionally, the optical calibration pattern (22) is arranged at the base (18) in a rigid manner relative to the external camera (16), and that a static first transformation (T1) between a position of the optical calibration pattern (22) and the position of the external camera (16) is stored in a storage unit (24) and is provided to the control unit (20) for determination of the position of the external camera (16), or
the external camera (16) is fastened on a base (18) and the optical calibration pattern (22) is relatively movable to the external camera (16) which tracks the relatively movable calibration pattern (22), in particular by way of the calibration tracker (28) which is mounted rigidly on the calibration pattern (22), with optical markers and a static seventh transformation (T7) to the calibration pattern (22) stored in a storage unit, wherein, based thereon, the control unit (20) calculates a dynamic first transformation (T1) from the position of the optical calibration pattern (22) to the position of the external camera (16).

10. The surgical assistance system (1) according to any of claims 8 to 10, **characterized in that** the external camera (16) is a stereo camera for tracking, more particularly an infrared-based stereo tracking camera, and the tracker (12) on the robot flange (6) is in particular an infrared-based tracker with a plurality of infrared markers spaced apart from each other.

11. The surgical assistance system (1) according to any of claims 8 to 10, **characterized in that** the robot flange (6), the tracker (12), and the robot camera (10) are rigid with respect to each other and in particular have a same distance to each other.

12. A computer-readable storage medium comprising instructions which, when executed by a computer, cause same to perform the method steps of the calibration method in accordance with any of claims 1 to 7.

## Revendications

1. Procédé d'étalonnage pour l'étalonnage automatisé d'une caméra de robot (10) sur un robot médical, en particulier chirurgical (2), et pour l'étalonnage automatisé d'un système de caméra externe avec au moins une caméra externe (16) sur le robot médical (2), dans lequel la caméra de robot (10) est fixée sur une bride de robot (6) d'un bras de robot (4) qui est relié à une base de robot (26), comprenant les étapes consistant à :
(a) déplacer la caméra de robot (10) au moyen du bras de robot (4) sous enregistrement (A) par la caméra de robot (10) ;
(b) détecter, sur la base de l'enregistrement (A), une position d'un motif d'étalonnage optique prédéterminé (22) et/ou d'un dispositif de suivi d'étalonnage (28) appliqué de manière fixe au motif d'étalonnage (22), respectivement avec une transformation prédéterminée de cette position détectée vers une position de la caméra externe (16), et/ou détecter, sur la base de l'enregistrement (A), une position de la caméra externe (16), dans lequel une première transformation prédéterminée (T1) représente la position du motif d'étalonnage optique prédéterminé (22) et/ou du dispositif de suivi d'étalonnage (28) par rapport à une position de la caméra externe (16) ;
(c) déterminer, sur la base de la position déterminée de la caméra externe (16), une deuxième transformation (T2) entre la caméra de robot (10) et la caméra externe (16) et déterminer un champ de vision de la caméra externe (16) ;
(d) déplacer la bride de robot (6) dans au moins trois positions différentes dans le champ de vision de la caméra externe (16) ;
(e) enregistrer les au moins trois positions de la bride de robot (6) par l'intermédiaire de la caméra externe (16) ;
(f) déterminer une troisième transformation (T3) de la bride de robot (6) vers la caméra de robot (10) et/ou une quatrième transformation (T4) de la caméra externe (16) vers la base de robot (26) ; et
(g) réaliser, sur la base des au moins trois positions détectées de la bride de robot (6) et d'au moins trois des première, deuxième, troisième ou quatrième transformations déterminées, un étalonnage main-œil.

2. Procédé d'étalonnage selon la revendication 1, **caractérisé en ce qu'**un dispositif de suivi (12) est fixé sur la bride de robot (6),
**en ce qu'**au moins trois positions du dispositif de suivi (12) sont détectées par la caméra externe (16),
**en ce qu'**une cinquième transformation (T5) de la bride de robot (6) vers le dispositif de suivi (12) est connue ou est déterminée sur la base des au moins trois positions détectées de la bride de robot (6) et du dispositif de suivi (12) fixé sur la bride de robot (6),
et **en ce qu'**un étalonnage main-œil est réalisé sur la base des au moins trois positions détectées de la bride de robot (6) et d'au moins trois des première, deuxième, troisième ou quatrième transformations déterminées ou de la cinquième transformation connue ou déterminée.

3. Procédé d'étalonnage selon la revendication 2, **caractérisé en ce que** les étapes de déplacement de la bride de robot et de réalisation de l'étalonnage œil-main du procédé d'étalonnage sont exécutées de manière itérative et, après un premier passage de la réalisation de l'étalonnage œil-main, la cinquième transformation connue ou déterminée (T5) entre le dispositif de suivi (12) et la bride de robot (6), une sixième transformation (T6) entre la caméra externe (16) et le dispositif de suivi (12) ainsi qu'une cinématique avant du robot (2) sont appliquées pour déterminer de nouvelles positions de la bride de robot (6) et déplacer la bride de robot (6) en conséquence dans ces positions pour l'itération suivante.

4. Procédé d'étalonnage selon l'une quelconque des revendications précédentes, **caractérisé en ce que** l'étape de déplacement de la caméra de robot (10) est réalisée sur la base de mouvements aléatoires jusqu'à ce que le motif d'étalonnage optique (22) ou le dispositif de suivi externe ou la caméra externe (16) soit détecté.

5. Procédé d'étalonnage selon l'une quelconque des revendications précédentes, **caractérisé en ce que** l'étape de détection du motif d'étalonnage optique (22) présente les étapes consistant à :
comparer des zones partielles de l'enregistrement (A) de la caméra de robot (10) avec un motif d'étalonnage enregistré (22) et, en cas de concordance : déterminer la position du motif d'étalonnage (22) au moyen d'une analyse d'image et déterminer, sur la base d'une première transformation enregistrée (T1) entre la position du motif d'étalonnage (22) et la position de la caméra externe (16), une position de la caméra externe (16).

6. Procédé d'étalonnage selon l'une quelconque des revendications précédentes, **caractérisé en ce que** l'étape de détection d'une position de la caméra externe (16) présente l'étape consistant à :
comparer des zones partielles de l'enregistrement (A) de la caméra de robot (10), en particulier d'un enregistrement 3D, avec un modèle géométrique enregistré, en particulier un modèle CAO, de la caméra externe (16) et, en cas de détection d'une concordance avec le modèle géométrique enregistré, déterminer une position de la caméra externe (16) par corrélation des structures tridimensionnelles.

7. Procédé d'étalonnage selon l'une quelconque des revendications précédentes, **caractérisé en ce que** le procédé d'étalonnage présente en outre l'étape d'un étalonnage géométrique, en particulier avant l'étape de déplacement de la caméra de robot (10) ou après l'étape de réalisation de l'étalonnage œil-main.

8. Système d'assistance chirurgical guidé (1) avec
au moins un robot (2) qui présente un bras de robot mobile (4) relié à une base de robot (26) avec une bride de robot (6) et en particulier un dispositif de suivi (12) sur la bride de robot (6) ;
une caméra de robot (10) reliée à la bride de robot (6), qui peut être déplacée au moyen du bras de robot (4) ; et
un motif d'étalonnage externe (22), ainsi qu'un dispositif de suivi d'étalonnage (28) appliqué de préférence sur le motif d'étalonnage (2),
un système de caméra externe (14) avec au moins une caméra externe (16), dans lequel la bride de robot (6), en particulier le dispositif de suivi (12) et/ou la caméra de robot (10), peut être déplacée dans un champ de vision de la caméra externe (16), **caractérisé en ce que** le système d'assistance (1) présente en outre une unité de commande (20) qui est adaptée pour réaliser le procédé d'étalonnage selon l'une quelconque des revendications précédentes 1 à 7.

9. Système d'assistance chirurgical (1) selon la revendication 8, **caractérisé en ce que**
la caméra externe (16) est fixée à une base (18) et, sur la base (18), le motif d'étalonnage optique (22) est en outre disposé de manière rigide par rapport à la caméra externe (16), et une première transformation statique (T1) entre une position du motif d'étalonnage optique (22) et la position de la caméra externe (16) est enregistrée dans une unité de stockage (24) et est fournie à l'unité de commande (20) pour déterminer la position de la caméra externe (16), ou
la caméra externe (16) est fixée à une base (18) et le motif d'étalonnage optique (22) est mobile par rapport à la caméra externe (16), laquelle suit le motif d'étalonnage mobile (22), en particulier par l'intermédiaire du dispositif de suivi d'étalonnage (28) fixé de manière rigide sur le motif d'étalonnage (22) avec des marqueurs optiques et une septième transformation statique (T7) enregistrée dans une unité de stockage par rapport au motif d'étalonnage (22), dans lequel l'unité de commande (20) calcule sur cette base une première transformation dynamique (T1) de la position du motif d'étalonnage optique (22) par rapport à la position de la caméra externe (16).

10. Système d'assistance chirurgical (1) selon la revendication 8 ou 9, **caractérisé en ce que** la caméra externe (16) est une caméra stéréo pour le suivi, en particulier une caméra de suivi stéréo à infrarouge, et le dispositif de suivi (12) sur la bride de robot (6) est en particulier un dispositif de suivi à infrarouge avec une pluralité de marqueurs infrarouges espacés les uns des autres.

11. Système d'assistance chirurgical (1) selon l'une quelconque des revendications 8 à 10, **caractérisé en ce que** la bride de robot (6), le dispositif de suivi (12) et la caméra de robot (10) sont rigides les uns par rapport aux autres et présentent en particulier un écart identique les uns par rapport aux autres.

12. Support de stockage lisible par ordinateur, qui comprend des instructions qui, lorsqu'elles sont exécutées par un ordinateur, amènent celui-ci à exécuter les étapes de procédé du procédé d'étalonnage selon l'une quelconque des revendications 1 à 7.
